# EUROPEAN PATENT APPLICATION

(11) **EP 2 835 640 A1**
(43) Date of publication of application: **11.02.2015**
(21) Application number: 13179500.7
(22) Date of filing: 06.08.2013
(51) Int. Cl.: G01N 33/50

(54) **Method for predicting the skin sensitizing activity of a compound.**

(71) Applicant: Academisch Ziekenhuis Leiden HODN Leids Universitair Medisch Centrum, 2333 ZA Leiden (NL)
(72) Inventor: Alloul-Ramdhani, Mariam, 2331 SZ Leiden (NL); Tensen, Cornelis, 2341 PN Oegstgeest (NL); Elghalbzouri, Abdelouahab, 2353 WZ Leiderdorp (NL)
(74) Representative: Habets, Winand

(57) **Abstract**

The invention relates generally to in vitro methods for evaluating the likelihood that a compound will act as a skin sensitizer in vivo. More in particular, the invention provides an in vitro method for predicting the in vivo skin sensitizing activity of a test compound comprising the steps of: Providing a cell culture of immortalised keratinocyte cells, contacting the cell culture with the test compound and measuring the expression level of at least the gene OSGIN-1, wherein it is concluded that the compound has in vivo skin sensitizing activity if the expression level of the gene OSGIN-1 is above a predetermined reference level.

SEQUENCE LISTING

<110>  ACADEMISCH ZIEKENHUIS LEIDEN HODN LEIDS UNIVERSITAIR
       MEDISCH CENTRUM

<120>  METHOD FOR PREDICTING THE SKIN SENSITIZING ACTIVITY OF A COMPOUND

<130>  274 EPP0

<160>  7

<170>  PatentIn version 3.5

<210>  1
<211>  23
<212>  DNA
<213>  homo sapiens

<400>  1
ttctcttggc tggcttcctt acc                                              23

<210>  2
<211>  33
<212>  DNA
<213>  homo sapiens

<400>  2
tataaagaca aaccaaagta aatctgaaag gag                                   33

<210>  3
<211>  21
<212>  DNA
<213>  homo sapiens

<400>  3
acctgcgtgt cctgtgctta c                                                21

<210>  4
<211>  19
<212>  DNA
<213>  homo sapiens

<400>  4
tgctgcagag gaagctcac                                                   19

<210>  5
<211>  21
<212>  DNA
<213>  homo sapiens

<400>  5
gatgagtatg cctgccgtgt g                                                21

<210>  6
<211>  22
<212>  DNA
<213>  homo sapiens

<400>  6
ctcatttgga attttgccga tt                                                22

<210>  7
<211>  20
<212>  DNA
<213>  homo sapiens

<400>  7
cacgaaccac ggcactgatt                                                   20

## Description

### Field of the invention

The invention relates generally to in vitro methods for evaluating the likelihood that a compound will act as a skin sensitizer in vivo.

### Background of the invention

Animal testing of chemical ingredients for cosmetic purposes is prohibited. Therefore, there is an urgent need for in vitro models to identify chemical allergens. In human skin, keratinocytes are abundantly present and are key players in initiation of allergic contact dermatitis.

Human skin is the first defence barrier against chemical insults and pathogens and consists for 90% out of keratinocytes. Several lines of evidence support the critical role for keratinocytes as key players in the early initiation of Allergic contact dermatitis (ACD) (Kaplan et al., 2012; Nestle et al., 2009; Nickoloff et al., 2006; Kollisch et al., 2005). ACD is a common occupational health problem, which results in a skin T-cell mediated inflammation caused by chemical sensitizers that make contact with the skin. The first phase of ACD is referred to as the sensitization phase, which does not lead to clinical manifestations. Chemical sensitizers act as haptens as they have the ability to bind skin-resident carrier proteins. Haptens can efficiently penetrate through the skin and make covalent bindings with carrier proteins. This hapten-protein complex is recognized by the innate immune system leading to production of pro-inflammatory mediators. As a consequence, effector T cells are mediated and enter the blood circulation. After re-exposure (second phase) of the hapten to the skin it causes an inflammation (Kimber et al., 2002; Cavani 2008; Gober & Gaspari 2008;Vocanson et al., 2009; Kaplan et al., 2012).

In order to guarantee the safety of compounds used in the chemical and cosmetic industry, it's essential to identify and predict the sensitizing capacity of these ingredients. Currently animal tests such as the local lymph node assay (LLNA) (Kimber et al., 1994, Basketter et. al., 2007) are used to identify chemical sensitizers and classify them according to their potency. Reducing, replacing and refinement of the use of animal experimentation in the cosmetic industry has led to the introduction of the EU 7th Amendment to the cosmetic directive and the implementation of the Registration Evaluation Authorization and Restriction of Chemicals (REACH) regulation. Although there are many promising tests in development, to date there are no in vitro test models yet validated by the European Centre for the Validation of Alternative methods (ECVAM). Therefore there is an increasing need for reliable in vitro models that are able to identify chemicals causing skin sensitization.

A significant body of data has emerged suggesting that the oxidative stress pathway is a key pathway induced by sensitizers. Sensor protein Keap1 (Kelch-like ECH-associated protein 1) is present as a complex with Nrf2 leading to its constitutive proteasomal degradation. Covalent binding of chemical allergens to Keap1 through activation of the reactive cysteine residues of Keap1, results into dissociation of the protein complex and stabilization of Nrf2 levels. Subsequently, Nrf2 translocates to the nucleus and induces transcription of genes containing an antioxidant response element (ARE) (Natsch & Emter 2008; vandeBriel 2010, Kaplan et al., 2012).

Several human cell lines have been used for skin sensitizer prediction (e.g. HaCat, MUTZ-3, THP-1, NCTC2455) either at transcription level or cytokine level. Mainly Nrf2 dependent genes were found to be activated by sensitizers in these cell lines (van Och et al., 2005, Ade et al., 2009, vandeBriel 2010, Corsini et al., 2009).

In another approach, human primary keratinocytes are used for the detection of sensitizers. Using human based reconstructed skin models for immune-toxic screening has major advantages as compared to conventional monolayer cell lines, since it consist of a competent barrier allowing topical application of chemicals with low aqueous solubility and finished (cosmetic) products. However, the generation of skin models is cumbersome and prone to experimental variations.

Nevertheless, most epidermal models are engineered using primary keratinocytes in order to mimic in vivo characteristics including a barrier function (Ponec et al., 1997; EI Ghalbzouri et al., 2008; Thakoersing et al., 2010).

There is an urgent need in the art to have more reproducible, reliable and sustainable biological systems for distinguishing sensitizers from irritants.

### Summary of the invention

Herein we demonstrate that immortalised keratinocyte cells are well suited for use in an in vitro test to distinguish irritants from sensitizers. , in particular a known cell line (N/TERT) provided good results. The human N/TERT cell line was obtained from J. Rheinwald laboratory, Harvard Medical School, Boston, USA. N/TERT is an hTERT-immortalized human keratinocyte cell line (Dickson et al., 2000).

This cell line provides all the advantages of a primary cell system in that it provides a barrier allowing the testing of compounds by topical applications whereas it also provides the sustainability and reproducibility of a cell line.

Moreover, the N/TERT epidermal model (NEM) show similar activation of the Keap1-Nrf2-ARE pathway as a primary keratinocyte model (the Leiden epidermal model (LEM), EI Ghalbzouri et. al., 2008, Boelsma et al., 2000; Ponec et al., 2000a,b, 2002).

We established that NEM is a promising screening tool to predict the sensitizing potential of chemicals and therefore may contribute to the reduction of animal experimentation. We also found that the expression of at least one gene (OSGIN-1) may distinguish whether or not a compound has skin sensitizing activity. We also found that the expression of OSGIN-1 may distinguish compounds with sensitizing activity from irritants.

The invention therefore relates to In vitro method for predicting the in vivo skin sensitizing activity of a test compound comprising the steps of:
a) Providing a cell culture of immortalised keratinocyte cells, preferably N/TERT cells,
b) Contacting the cell culture with the test compound,
c) Measuring the expression level of at least the gene OSGIN-1,
wherein it is concluded that the compound has in vivo skin sensitizing activity if the expression level of the gene OSGIN-1 is above a predetermined reference level.

### Detailed description of the invention

In the study that led to the present invention, we developed a keratinocyte epidermis model based on human N/TERT cells that was able to distinguish between sensitizers and irritants.

A key step in skin sensitization is the covalent binding of the sensitizer to endogenous proteins in the skin and thereby inducing the production of reactive oxygen species (ROS) by a yet unknown mechanism. In addition, tests based on ROS production are in development, such as the THP-1 cell line showing the ability to distinguish sensitizers from irritants (Esser et al., 2012, Saito et al., 2013). Cells induce a danger signal in response to ROS by activating the Keap1-Nrf2-ARE pathway. Natsch and colleagues used the Keap1-Nrf2-ARE pathway as a reporter assay incorporated in a commercial HaCat keratinocyte cell line, the Keratinosens®, which is based on transcriptional activation of Nrf2 targets (Natsch and Emter 2008, McKim et al., 2010-2012, van der Veen 2012 Vandebriel 2010). We now found that this pathway is induced in primary keratinocyte cells as well as in the N/TERT cell line upon sensitization.

Commercially available primary human reconstructed epidermal models have been previously used in order to discriminate sensitizers from irritants (McKim et al., 2012). However, it is known that donor variability of primary cell culture models cause quite some variability and therefore data can be difficult to interpret. Moreover, human skin needed for construction of primary human skin equivalents is often difficult to obtain.In order to minimize the donor variability we herein propose the use of an epidermal model derived from a cell line.

We tested the N/TERT cell line for its ability to generate an epidermal model consisting of a prober barrier with similar properties compared to the previously described model consisting of primary keratinocyte cells termed LEMs (EI Ghalbzouri et. al., 2008, Boelsma et al., 2000; Ponec et al., 2000a,b, 2002).

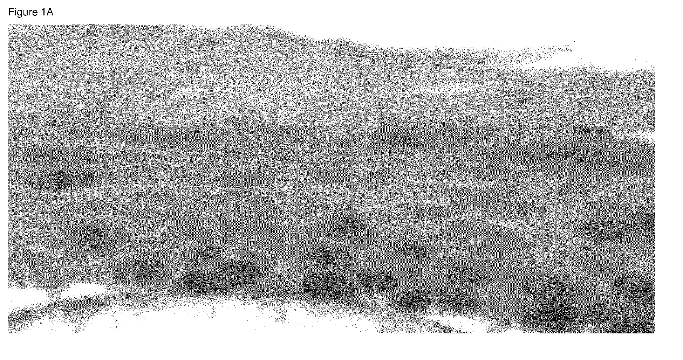

Morphological analyses and epidermal morphogenesis demonstrated a high similarity between LEMs and NEMs (figure 1a). (Thakoersing et. al., 2012). We found that the N/TERT keratinocyte cell line may be used to generate an epidermal model with a competent barrier, which is comparable to the LEM model.

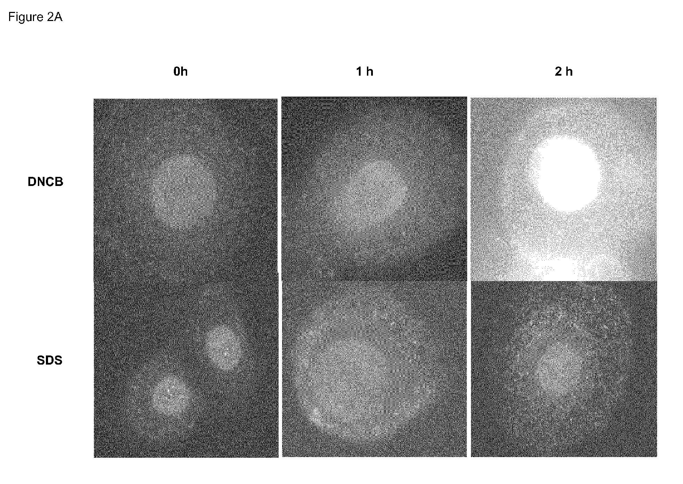

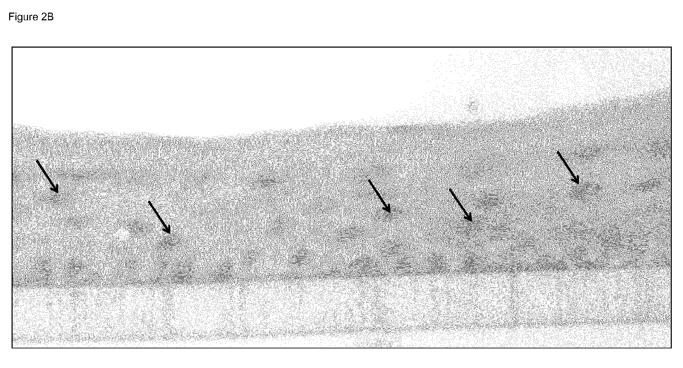

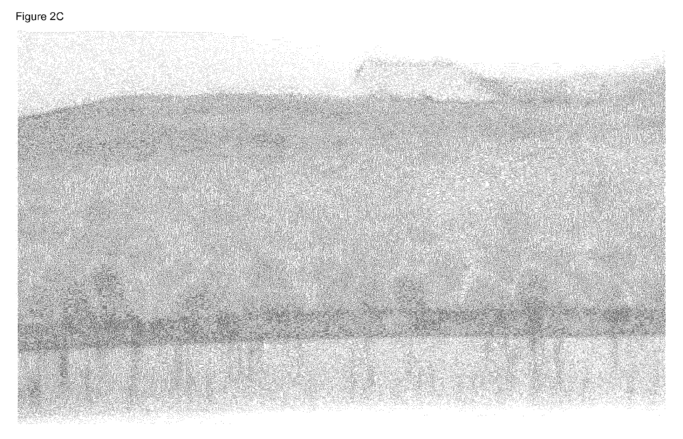

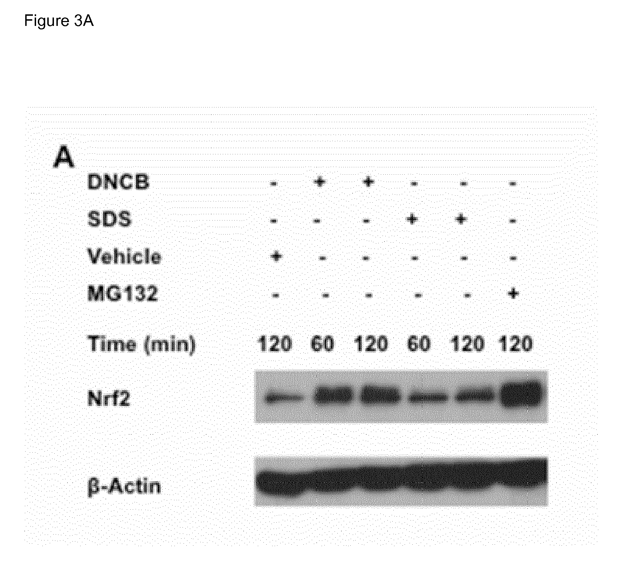

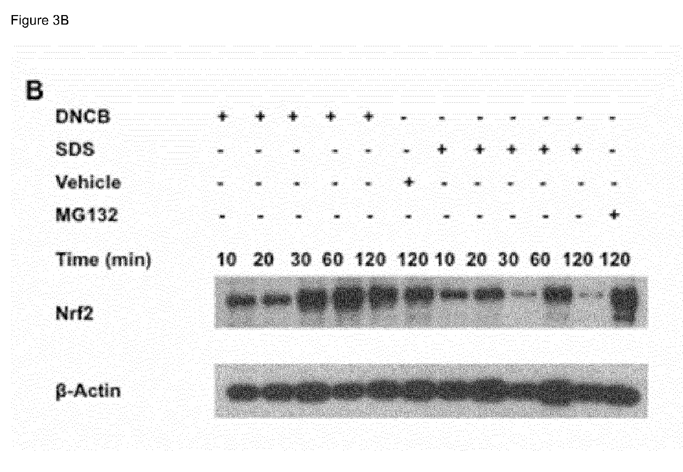

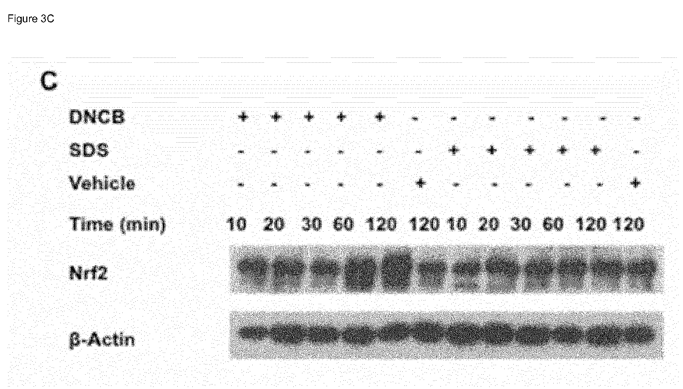

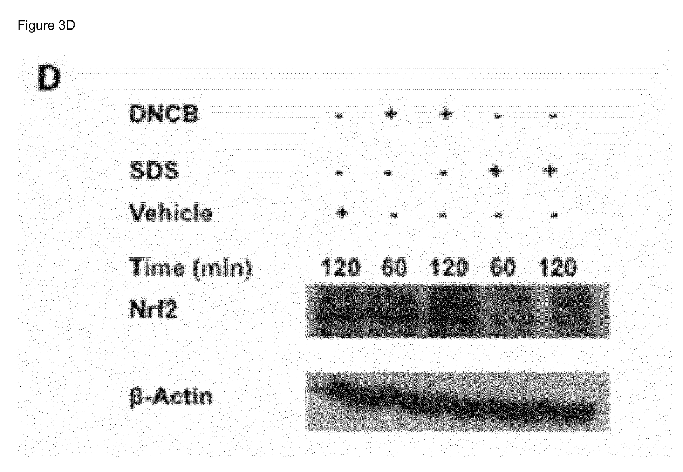

In the study described herein, we use DNCB as a sensitizer and irritant SDS as a control at non-toxic concentrations. We show that Nrf2 is translocated after sensitization in monolayer keratinocytes, but more importantly also in NEMs. Immunohistochemical staining as well as western blot analysis were used to show Nrf2 accumulation and translocation to the nucleus (Figure 2 and 3) indicating activation of Keap1-Nrf2-ARE pathway.

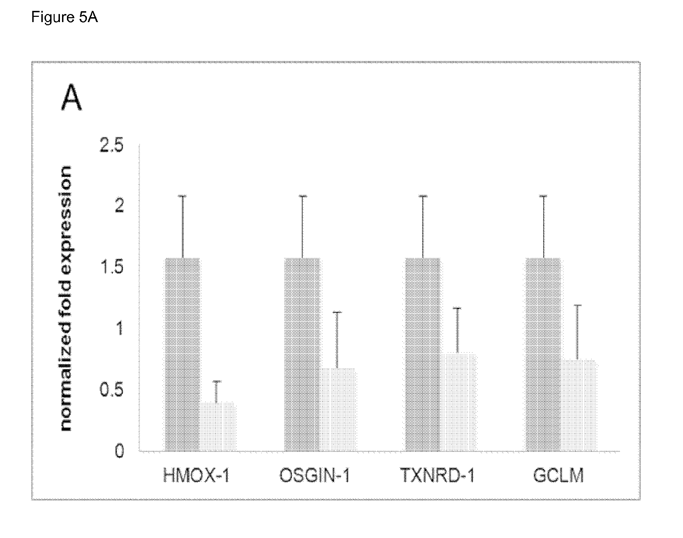

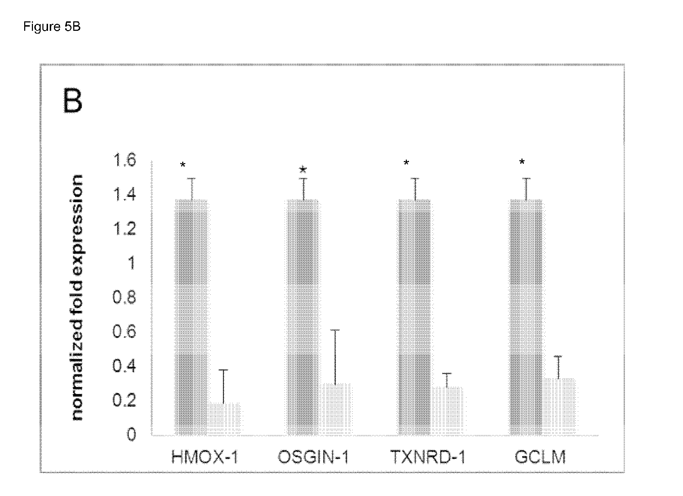

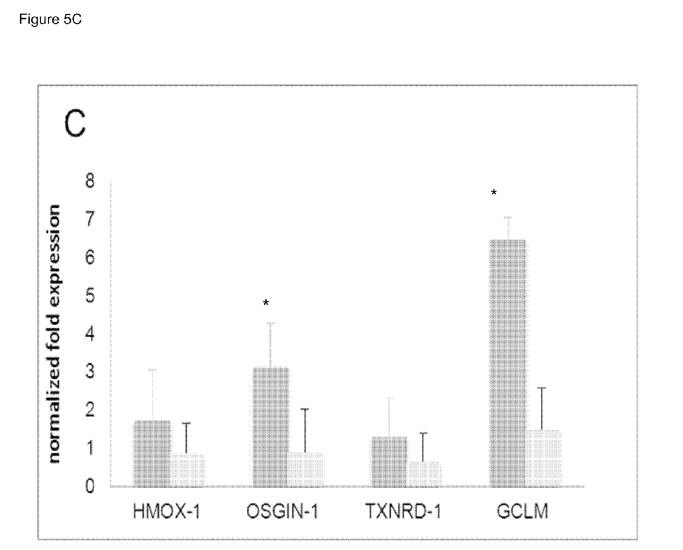

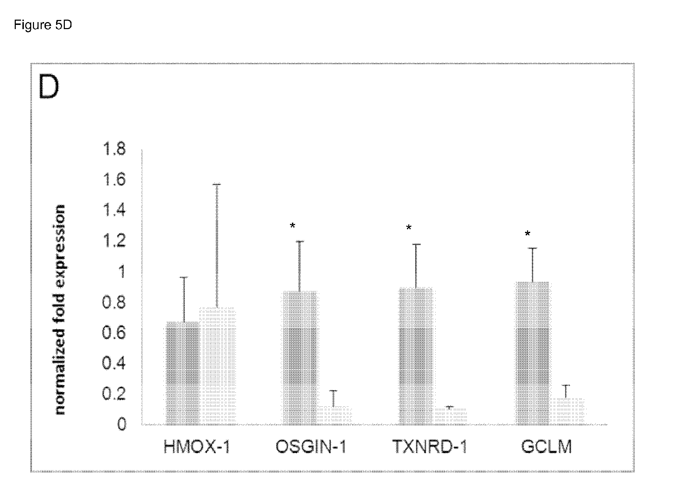

Transcriptional activity of transcription factor Nrf2 of a subset of its targets genes was measured by quantitative PCR after 4 h exposure (figure 5). We selected four Nrf2 targets HMOX-1, TXRND-1, OSGIN-1 and GCLM (Ade et al., 2009, Natsch&Emter 2008, VandeBriel 2010,Van der Veen et al., 2012). HMOX-1 is previously described to be significantly upregulated at mRNA expression levels after chemical sensitization in several different monolayer cell lines, such as THP-1 cells, Dendritic cells and HaCat cells (Ade et al., 2009, VandeBriel 2010,Van der Veen et al., 2012). We found HMOX-1 to be differentially upregulated in monolayer primary KCs and N/TERTs. However, we could neither observe any significant up regulation of the HMOX-1 gene in NEMs, nor in ex-vivo sensitized skin after exposure with sensitizer DNCB.

Moreover staining for HMOX-1 protein levels in both LEMs and NEMs showed constitutive expression of HMOX-1 in the granular layer (data not shown), which might suggest an altered regulatory mechanism for HMOX-1 at transcriptional levels in a differentiated state. TXRND-1 was significantly upregulated in N/TERT monolayers and ex-vivo skin but not significant in primary keratinocytes and LEMS. OSGIN-1 and GCLM were significantly upregulated after sensitization irrespective the model used. We therefore focussed on OSGIN-1 and GCLM as potential biomarkers for identification of sensitizers in NEMs.

The terms LEMs and NEMs as used herein refer to a stratified keratinocyte layer that contains a competent barrier originating from either an immortalized cell culture or from primary monolayer cells. Stratified layers originating from primary cells are termed LEMs whereas stratified layers originating from immortalized cells are called NEMs.

N/TERT cells may be used as a source to obtain NEMs. For that purpose, the cells are seeded onto a filter insert or membrane of tissue culture trays and cultured submerged until confluence has been reached (80-100%). After two days of submerged culture, cells are exposed to the air for 7-14 days to induce differentiation until a stratified keratinocyte layer has been formed that contains a competent barrier. The optimal use for toxicity screening is between 10 and 12 days of air exposure.

The invention therefore relates to an vitro method for predicting the in vivo skin sensitizing activity of a test compound comprising the steps of:
a) Providing a cell culture of N/TERT cells,
b) Contacting the cell culture with the test compound,
c) Measuring the expression level of at least the gene OSGIN-1,
wherein it is concluded that the compound has in vivo skin sensitizing activity if the expression level of the gene OSGIN-1 is above a predetermined reference level.

The skilled person is well aware of methods available in the art for determining the expression levels of marker genes such as OSGIN-1 or GCLM. This may be performed using a microarray or by sequencing, however a preferred method is quantitative PCR. A particularly suited method for determining the expression level of OSGIN-1 or GCLM is exemplified herein in the following examples.

N/TERT cells may be used as monolayers or in a three-dimensional model for skin epidermus (NEM).

The invention therefore also relates to a method as described above wherein the N/TERT cells are comprised in astratified keratinocyte layer which contains a competent barrier

A second marker gene GCLM may also advantageously employed in a method as described above.

Hence, the invention also relates to a method as described above wherein also the expression level of the gene GCLM is determined and wherein it is concluded that the compound has in vivo skin sensitizing activity if the expression level of the genes OSGIN-1 and GCLM are above a predetermined reference level.

A skilled person is well aware of the way in that a reference value is determined. This may done in several ways that all may be advantageous for a given method. For example, the method according to the invention may be performed wherein the test compound is replaced with a control compound. That control compound may be an non-sensitizing compound, an irritant or a composition comprising the carrier in which the test compound is provided.

Hence, in a preferred embodiment, the invention relates to a method as described above wherein the predetermined reference level is the level of expression of the genes OSGIN-1 or GCLM when the cell culture is exposed to an irritant. A particularly preferred control compound is SDS or DCB. Hence, the invention relates to a method as described above wherein the irritant is SDS or DCB.

### Legend to the figures.

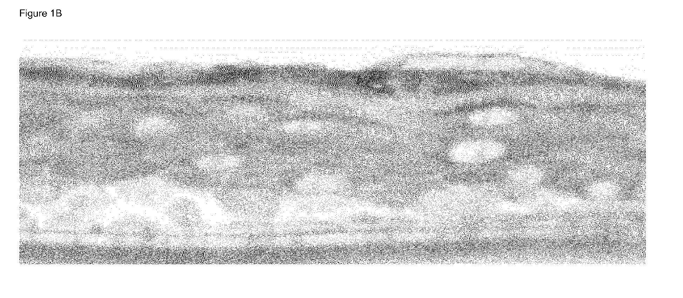

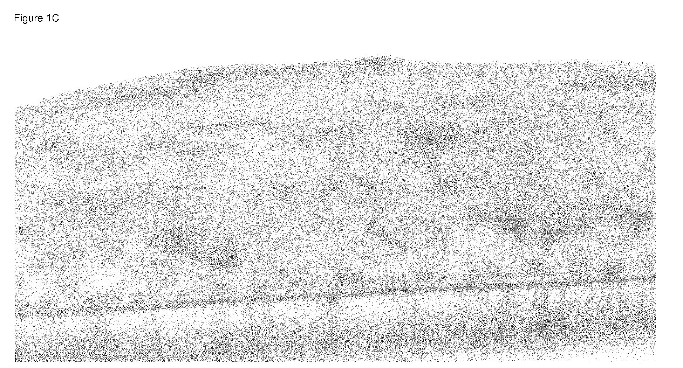

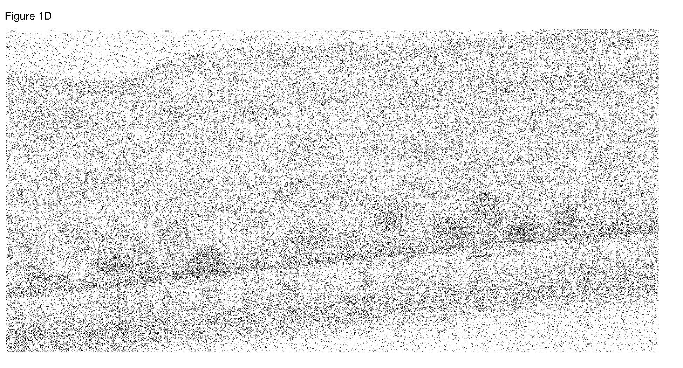

Figure 1: Morphology and differentiation of NEMs. (A) Shown are cross sections of a Haematoxylin and Eosin (HE) staining on NEMs that have been cultured for 12 days at the air liquid interface. (B) Immunohistochemical staining showing the presence of K10 in the suprabasal layers and a very weak expression of the hyper-proliferation associated marker K16 (C) in the upper layers. Cells positive for the proliferation marker Ki67 are detected in the basal layer (arrows) (D). Scale bar represents 25 µm.
Figure 2: Nrf2 nuclear translocation after DNCB exposure of keratinocytes. (A) Monoculture of primary keratinocytes were treated with either DNCB (sensitizer) or SDS (irritant) for 0, 1 or 2 h. Immunofluorescence staining was performed in order to detect Nrf2 using Rhodamine (red). Nuclei were visualized using DAPI staining (blue). Nuclear translocation of Nrf2 was clearly visible after treatment with DNCB (see asterisk). NEMs cultured for 12 days air-exposed were treated with DNCB (B), and with SDS (C) for 4 h. Nrf2 was only translocated to the nucleus after DNCB treatment. Scale bar represents 50 µm.
Figure 3: Nuclear fractionation of keratinocytes after treatment with DNCD and SDS. Monolayer of primary keratinocytes (A) and monolayer of the N/TERT cell line (B) were treated at different time points with either DNCB or SDS. As a negative control cells were treated with vehicles and as a positive control the proteasome inhibitor MG132 was used. LEMs (C) and NEMs (D) were treated in the same way. Nuclear lysates were isolated and subjected to Western blot analysis in order to detect Nrf2 protein levels. Enhanced levels of Nrf2 were detected in all DNCB treated keratinocytes and epidermal models. In all experiments b-actin was used as a loading control. Results represent three independent experiments.

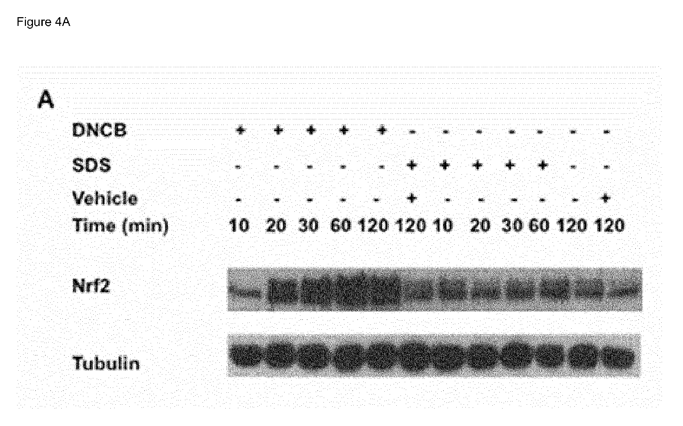

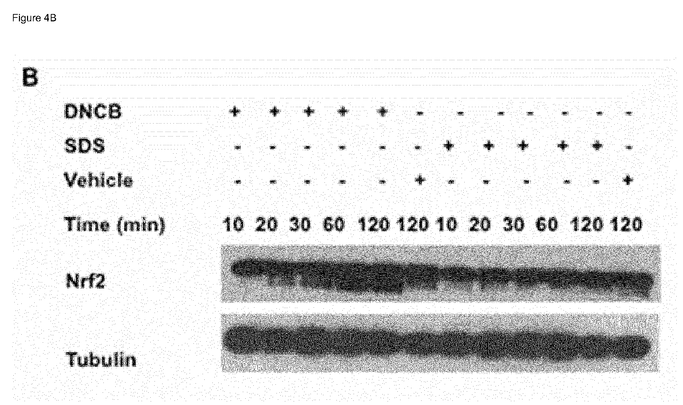

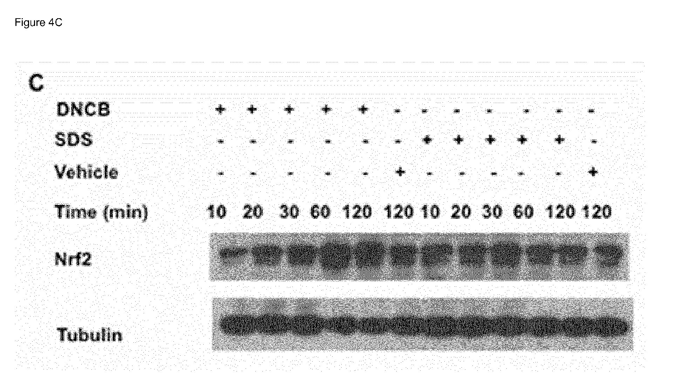

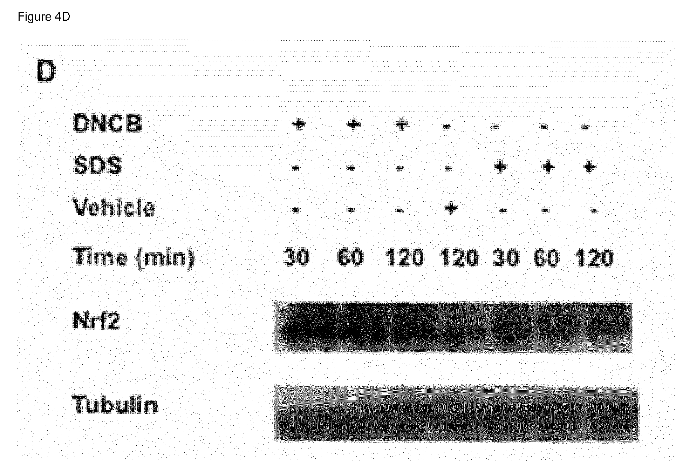

Figure 4: Nrf2 protein expression levels in primary keratinocytes and the N/TERT cell line.
   Monolayer of primary keratinocytes (A) and monolayer of the N/TERT cell line (B) were treated at different time points with either DNCB or SDS. Vehicle treatment served as a negative control. LEMs (C) and NEMs (D) were treated with either DNCB or SDS in a time dependent manner. Total lysates were prepared after chemical exposure and subjected to Western blot analysis in order to detect Nrf2 protein levels. Enhanced levels of Nrf2 were detected in all DNCB treated keratinocytes and epidermal models. In all experiments tubulin was used as a loading control. Results represent three independent experiments
Figure 5: Elevated mRNA expression of Nrf2 dependant genes after treatment with DNCB or SDS.
   Monolayer of primary keratinocytes (A); monolayer of the N/TERT cell line (B); NEMs (C); and ex-vivo epidermal sheets (D). All different models were treated with DNCB or SDS for 4 h. Q-PCR analysis was performed and gene expression was normalized using GUSB, B2M and TBP as reference genes. Error bar show SEM. *P<0.05. Results represent three independent experiments.

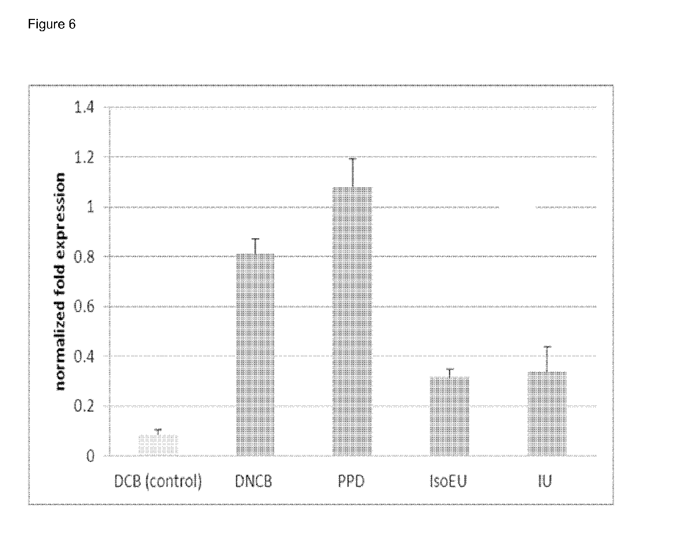

Figure 6: Expression levels of OSGIN-1 correlate with potency levels of sensitizers. NEMs were exposed to 4 different sensitizers at concentrations of 80 - 85% CV: DNCB (0.5 mM), PPD (35 mM), IsoEu (15 mM) and IU (30 mM). DCB, an irritant (3mM) was used as control.

### Examples

### Example 1: Primary human keratinocytes.

Primary human keratinocytes (KCs) were isolated from human mammary skin after informed consent according to the principles and guidelines of the Declaration of Helsinki, as described earlier (Ponec et al., 1997, EI Ghalbzouri et al., 2008). In short, fresh skin tissue was obtained from surgical mamma reductions. First the epidermis is enzymatically separated from the dermis overnight using dispase II (Roche Diagnostics, Mannheim, Germany). The next day KCs were isolated from the epidermis using trypsin for 15 min at 37 °C followed by trypsin inactivation. Cells were filtered using a 70 µm cell strainer (BD Biosciences, Breda, The Netherlands) and cultured in keratinocyte medium at 37 °C and 7.3% CO2. Primary KCs were cultured in DMEM/Ham's F12 medium (ratio 3:1) supplemented with 5% HyClone calf serum (Greiner, Nürtingen, Germany), 0.5 µM hydrocortisone, 1 µM isoproterenol, 0.1 µM insulin (Sigma), 100 U/ml penicillin and 100 µg/ml streptomycin (Invitrogen, Breda, the Netherlands). In all experiments secondary cultures were used. Cultures were refreshed every two days. These cells were used to construct the LEMS.

### Example 2: N/TERT keratinocyte cell line

Human N/TERT cell line were obtained fromJ. Rheinwald laboratory, Harvard Medical School, Boston, USA. N/TERT is an hTERT-immortalized human keratinocyte cell line (Dickson et al., 2000). Cells were cultured at 37 °C and 7.3% CO2 in keratinocyte-serum free medium (K-SFM) supplemented with the following final concentrations; 25ug/ml Bovine pituitary extract (BPE), 0.4 mM CaCl2, 0.2 ng/ml hEGF, 100 U/ml penicillin and 100 µg/ml streptomycin (Invitrogen, Breda, the Netherlands). Cultures were refreshed every two days. These cells were used to construct the NEMs.

### Example 3: Reconstructed epidermis

The LEMs and the NEMs were constructed by seeding 200.000 cells on insert filters (12 wells plate, polyester membrane, Costar) in Dermalife K medium including lifefactors (Lifeline Cell Technology, Walkersville, MD) until full density was reached. Dermalife medium was supplemented with 10 µM I-carnitine (Sigma), 10 mM I-serine (Sigma), 1 µM hydrocortisone, 1 µM isoproterenol, 0.1 µM insulin, 53 µM selenious acid (Sigma), 100 U/ml penicillin and 100 µg/ml streptomycin (Invitrogen). Thereafter they were cultured in CNT medium (basal medium plus supplement kit, CellnTec, Bern, Switzerland). This medium was supplemented with 2.4 x 10-5 M bovine serum albumin, 25µM palmitic acid, 15µM linoleic acid and 7 µM arachidonic acid (Sigma) Next, cultures were lifted to air/liquid interface and after one day the linoleic acid concentration was increased to 30 µM. Medium was refreshed every 2 days. LEMs and NEMs were air-exposed for 12 days at 37 °C and 7.3% CO2 prior to analysis.

### Example 4: Morphology and immunohistochemistry.

LEMs and NEMs sections were washed in phosphate-buffered saline (PBS), fixed with 4% formaldehyde and paraffin-embedded. Sections were cut at 5 µm thickness, deparaffinized, rehydrated, and stained with haematoxylin and eosin (HE). Immunohistochemical analysis was performed for Keratins 10, 16 and Ki67. Heat-mediated antigen retrieval was performed in citrate buffer pH6 followed by blocking non-specific binding using PBS containing 1% bovine serum albumin (BSA, Sigma) and 2% normal human serum (NHS, Sanquin, Leiden, the Netherlands). Primary antibodies were diluted in PBS+1%BSA+2%NHS and incubated overnight at 4 °C). After washing with PBS sections were incubated with streptavidin-biotin-peroxidase complex system (GE Healthcare, Buckinghamshire, UK) according to the manufacturer's protocol. After washing in PBS, the signal was developed with 3, 3'-diaminobenzidine (DAB) solution, and all slides were counterstained with haematoxylin. As negative controls, tissue sections were processed under the same experimental conditions as described above, except that they were incubated overnight at 4°C in PBS+1%BSA+2%NHS without primary antibody.

### Example 5: Immunofluorescence

Cells were grown on glass coverslips and exposed to irritants or sensitizers at CV concentrations between 80 and 100%. Cells were fixed after 24 h with 4% formaldehyde for 15 min at room temperature (all of the following steps were done at room temperature) and permeabilized in 0.2% Triton X-100-phosphate-buffered saline for 5 min. Cells were washed with phosphate-buffered saline and blocked with 5% goat serum for 1 h, incubated with primary antibodies for 1 h, washed, and incubated with secondary antibodies for 30 min, following extensive washing. All sections were counterstained with 4',6-diamidino-2-phenylindole, dihydrochloride (DAPI; Molecular Probes, Invitrogen) for 2 min. Sections were mounted with Vectashield (Brunschwig chemie, Burlingame, USA), covered and visualized with a Leica DM5000 B microscope.

### Example 6: WST proliferation assay

Cell viability of monolayers was measured using WST-1 assay (Roche diagnostics, Mannheim, Germany). Briefly, cells were seeded in 96-well flat-bottom plates (Greiner Bio-One, Frickenhausen, Germany) in 100 µl of culture medium 24-48 h prior to chemical treatment. After 24 h of treatment with an irritant or sensitizer, 10 µl of WST-1 reagent was added and plates were further incubated up to 3 h at 37°C. Absorbance was measured at 450 nm using a plate reader (TECAN infinite F200, Tecan, Austria) in order to calculate the cell viability. Monolayer keratinocytes were exposed to DNCB (10 µM) and SDS (80µM) at 80% cell viability concentrations.

### Example7: MTT proliferation assay

Twelve days air-exposed LEMs or NEMs were covered with a 11 mm filter paper disc (Finn Chamber, Phoenix, USA). Chemicals with different doses were applied onto the filters and incubated for 24 h and thereafter washed away with PBS. LEMs or NEMs were then incubated with thiazolyl blue tetrazolium bromide (MTT), (Sigma). MTT stock of 5 mg/ml PBS (filter sterilized) was diluted to a final concentration of 1 mg/ml in colourless DMEM medium. MTT medium was added to the cell cultures and incubated for 3 h at 37°C. Viable cells were then allowed to reduce the tetrazolium dye to its insoluble formazan, which has a purple colour. Therefore, after 3 hours, the MTT was removed and washed away with PBS. After drying, the formazan was dissolved in 2ml of isopropanol overnight at room temperature. Absorbance was measured at 570 nm using a plate reader (TECAN infinite F200) in order to calculate the cell viability.

### Example 8: Nuclear fractionation assay

Nuclear fractions of monolayer cultures and reconstructed epidermis were isolate using a Nuclear extract kit (Active motif, Carlsbad, CA) according to the manufacturer's protocol. Nuclear fractions were subjected to Western blot analysis.

### Example 9: Western blot analysis

Monolayer cells were lysed in M-Per buffer (Thermo Scientific, Rockford, USA), reconstructed epidermis was cut in pieces and lysed in T-Per lysis buffer (Thermo Scientific, Rockford, USA) both supplemented with protease inhibitor cocktail EDTA-free and HALTTM phosphatase inhibitors (Thermo Scientific). Cell lysates were measured using the BCATM protein assay kit (Thermo Scientific). Equal amounts of proteins were separated by SDS-page. Proteins were transferred onto PVDF- transfer membranes (Thermo Scientific). Membranes were blocked in PBS, 0.1% Tween-20 (PBST) containing non-fat dry milk (ELK, Campina, The Netherlands) and subsequently incubated with the appropriate primary antibody. Membranes were washed four times with PBST and incubated with either horseradish peroxidase-conjugated goat anti-mouse or goat anti-rabbit secondary antibodies (1:2500, Pierce Thermo Scientific, Rockford, USA). After four times washing with PBST, proteins were detected by enhanced chemi-luminescence (Super signal West femto (Pierce Thermo Scientific, Rockford, USA) and visualized by exposure to X-ray film (Fujifilm Europe GmbH, Düsseldorf, Germany).

### Example 10: Analysis of gene expression by quantitative PCR

Total RNA was prepared using RNeasy mini kit (Qiagen) according to the manufacturer's protocol. RNA was quantified using a Nanodrop ND-1000 spectrophotometer. First- strand cDNA synthesis was derived from 1 ug of total RNA using the iscript cDNA synthesis kit (Biorad) according to the manufacturer's protocol. Primers were tested in normal KC cDNA. Stability of reference genes was analyzed with the GeNorm method (Vandesompele et al., 2002). Expression analysis was performed with the BioRad software (iQ5) and was based on the delta delta Ct method with the reference genes that were most stably expressed according to the GeNorm analysis.

Expression levels of the following genes were measured: HMOX-1 (Hemeoxygenase-1), GCLM (Glutamate-cysteine ligase modifier subunit), TXNRD1 (Thioredoxin reductase 1), OSGIN-1 (oxidative stress induced growth inhibitor 1), B2M (Beta-2 microglobulin), GUSB (Glucuronidase beta) and TBP (TATA binding protein). Quantitative real-time PCR was determined by using the following primer sets:
5'-TTCTCTTGGCTGGCTTCCTTACC-'3 (HMOX-1),
5'-TATAAAGACAAACCAAAGTAAATCTGAAAGGAG-'3 GCLM),
5'-ACCTGCGTGTCCTGTGCTTAC-'3 (TXNRD1),
5'- TGCTGCAGAGGAAGCTCAC-'3 (OSGIN1),
5'-GATGAGTATGCCTGCCGTGTG-'3 (B2M),
5'-CTCATTTGGAATTTTGCCGATT-'3 (GUSB),
5'-CACGAACCACGGCACTGATT-'3 (TBP).

Real-Time PCRs were performed in 10 µl reaction volumes using the MylQ system and SYBR Green PCR Master- mix (Biorad) according to the manufacturer's protocol.

The PCR cycles were: 3 minutes at 95 °C to activate the polymerase, 40 cycles of 20 seconds at 95 °C and 40 seconds at 60 °C, followed by the generation of a melt curve.

All qPCR values were averaged relative to the three different reference genes: B2M, GUSB, TBP. For each data point, PCRs were performed in duplicate and values represent means ± standard deviation.

### Example 11: Statistical analyses

Data were analyzed for statistical significance using the student t-test. P values of ≤ 0.05 were considered significant.

### Example 12: Antibodies, drugs and chemicals

The following antibodies were used: anti-Nrf2 rabbit polyclonal, 1:500 and 1:100 (Santa Cruz, CA, USA), anti-Tubulin rabbit polyoclonal, 1:2000 (Abcam, Cambridge, UK), anti-B-actin rabbit polyclonal, 1:2000 (Cell Signaling, MA, USA). Secondary antibodies: Rhodamine RedTM-X-conjugated goat anti-Rabbit 1:300 (Jackson Immunoresearch Europe, Suffolk, UK). Drugs: MG132 (Calbiochem) was used as a proteosome inhibitor for 6 h prior to lysis at a concentration of 3 µM. Chemicals: 2,4-dinitrochlorobenzene (DNCB) and Sodium Dodecyl Sulfate (SDS) were purchased from Sigma.

### Example 13: NEMs show similar epidermal morphogenesis as LEMs

To evaluate whether the N/TERT cell line epidermis model (NEMs) show comparable characteristics as the primary keratinocytes of the LEM model, we have examined morphology, the expression of the early differentiation marker Keratin 10 (K10), hyper-proliferation associated marker K16, and epidermal proliferation by Ki-67. As shown in figure 1A, the epidermis generated with N/TERT cell line show all different layers (the stratum basale, stratum spinosum, stratum granulosum and the stratum corneum) as found in LEMs and native human skin (data not shown). The expression of K10 is present in all suprabasal cell layers and negative in the basal layers, indicating the presence of a normal early differentiation program (Figure 1 B). The epidermis also shows a very weak expression of the hyperproliferation-associated marker K16 in the upper layers of the epidermis (Figure 1 C), indicating a normalized epidermis. As in LEMs and native skin, NEMs show comparable presence of proliferating basal keratinocytes (Figure 1D) (15±5 Ki67 positive cell/100 basal keratinocytes).

### Example 14: Nrf2 translocation occurs in primary keratinocytes and in the N/TERT cell line

After having demonstrated that morphological characteristics in NEMs and LEMS are similar, we investigated how both cell types in monolayer cultures and reconstructed epidermis respond on chemical sensitizers. For this purpose cell cultures of both N/TERT and primary keratinocytes were treated without (t=0) or with 2, 4-dinitrochlorobenzene (DNCB, 1.25 mM), a strong skin sensitizer) and Sodium dodecyl sulphate (SDS, 10 mM), a skin irritant, as a negative control for 1 or 2 h.

We found that after treatment of monolayer keratinocytes with DNCB a substantial amount of Rhodamine labelled Nrf2 protein was translocated to the nucleus compared to cells treated with SDS (Figure 2A). Furthermore immunohistochemical analyses of NEMs treated with DNCB (Figure 2B) also showed higher levels of Nrf2 in the nuclei compared to SDS treated epidermal models (Figure 2C). Similar results were obtained with the LEMs (data not shown).

Next we investigated whether Nrf2 expression could be stabilized by inhibiting the proteasome that is responsible for a constitutive degradation of Nrf2. We therefore used proteasome inhibitor MG132 as a positive control for Nrf2 translocation. Although the Nrf2 translocation was clearly visible, we used a second approach enabling quantification of the amount of translocated Nrf2. For this purpose, we prepared nuclear extracts of monolayer cells cultures and epidermal models and subjected them to Western blot analysis in order to detect translocated Nrf2 protein levels in the nuclei (Figure 3). As shown in figure 3A, we clearly observe elevated levels of Nrf2 in monolayer primary keratinocytes treated with DNCB and MG132 as well as in N/TERT monolayer cultures (figure3B). We could also confirm this in the LEMs (figure 3C) and NEMs (figure 3D). These results show that Nrf2 is prevented from degradation after DNCB treatment.

### Example 15: Elevated expression of total protein levels of Nrf2 in primary keratinocytes and the N/TERT cell line.

We also determined the total protein levels of Nrf2 in monolayer cultures of primary and N/TERT keratinocytes as well as the NEMs and LEMS. Western blot analysis was performed on all four keratinocyte-based models in order to detect endogenous levels of Nrf2. We observe an elevated protein expression of Nrf2 after DNCB treatment compared to SDS treated cells in a time dependent manner (Figure 4). When comparing monolayer primary keratinocytes (figure 4a) and monolayer N/TERT (figure 4b) we demonstrate that in both cell types the Nrf2 levels are induced when treated with DNCB. LEMs (figure 4c) and NEMs (figure 4d) also showed increased levels of Nrf2 after DNCB treatment. These results indicate that the sensitizer DNCB at least prevent Nrf2 degradation and/or enhances its expression levels.

### Example 16: Elevated mRNA expression levels of Nrf2 depended genes after sensitization.

We also explored the transcriptional activity of Nrf2. It is known that genes that have an ARE consensus in their promoter region can be activated by transcription factor Nrf2. We have therefore selected a subset of genes (HMOX-1, OSGIN-1, TXRND-1 and GCLM) that are described in literature to be transcriptional activated by Nrf2 in order to evaluate their mRNA levels after treatment with either DNCB or SDS. First we compared the activation of these genes in monocultures of primary and N/TERT keratinocytes (figure 5a and b). Expression of HMOX-1, OSGIN-1 and GCLM was significantly upregulated (approx. 1,2 fold) in primary keratinocytes (figure 5A), while in N/TERT monolayer cultures HMOX-1, OSGIN-1, TXNRD-1 and GCLM were significantly upregulated (approx.1,2 fold) (figure 5B). In addition, the same set of genes was also tested under the same conditions in NEMs and ex-vivo human skin (figure 5C and 5D). GLCM (approx. 3,2 fold) and OSGIN-1 (approx. 2 fold) were significantly upregulated in NEMs in response to DNCB (figure 5c). However, when analyzing the genes in ex-vivo skin we could measure elevated mRNA expression of OSGIN-1, TXRND-1 and GCLM, while HMOX-1 expression was not upregulated (figure 5D). This result indicates that most tested Nrf2 depended genes are significantly activated in the monolayer and epidermal models after sensitization compared to the irritant SDS.

### Example 17: Prediction of potency category.

In addition, we were able to show that the method according to the invention was also able to predict the potency category of the skin-sensitizing compound. We therefore tested four sensitizers with different potency in a method according to the invention. The results obtained with DNCB (extreme sensitizing) PPD (strong), IsoEu (moderate) and IU (weak) are shown in figure 6.

| Chemical | Abbreviation | Potency category |
|---|---|---|
| 2,4-Dinitrochlorobenzene | DNCB | Extreme |
| p-Phenylenediamine | PPD | Strong |
| Isoeugenol | IsoEU | moderate |
| Imidazolidinyl urea | IU | weak |

It may be concluded from this that extreme and strong sensitizers induce significantly higher expression levels of OSGIN-1 than moderate or weak sensitizers.

### References

Ade, N., Leon, F., Pallardy, M., Peiffer, J.L., Kerdine-Romer, S., Tissier, M.H., Bonnet, P.A., Fabre, I., Ourlin, J.C., 2009. HMOX1 and NQO1 genes are upregulated in response to contact sensitizers in dendritic cells and THP-1 cell line: role of the Keap1/Nrf2 pathway. Toxicol Sci 107, 451-60.
Basketter, D.A., Gerberick, F., Kimber, I., 2007. The local lymph node assay and the assessment of relative potency: status of validation. Contact Dermatitis. 57, 70-75.
Boelsma, E., Gibbs, S., Faller, C., Ponec, M., 2000. Characterization and comparison of reconstructed skin models: morphological and immunohistochemical evaluation. Acta Derm. Venereol. 80, 82-88.
Cavani, A., 2008. Immune regulatory mechanisms in allergic contact dermatitis and contact sensitization. Chem Immunol Allergy. 94, 93-100
Centanni, J.M., Straseski, J.A., Wicks, A., Hank, J.A., Rasmussen, C.A., Lokuta, M.A., Schurr, M.J., Foster, K.N., Faucher, L.D., Caruso, D.M., Comer, A.R., Allen-Hoffmann, B.L., 2011. StrataGraft skin substitute is well-tolerated and is not acutely immunogenic in patients with traumatic wounds: results from a prospective, randomized, controlled dose escalation trial. Ann Surg. 253, 672-83.
Corsini, E., Mitjans, M., Galbiati, V., Lucchi, L., Galli, C.L., Marinovich, M., 2009. Use of IL-18 production in a human keratinocyte cell line to discriminate contact sensitizers from irritants and low molecular weight respiratory allergens. Toxicol. In Vitro. 23, 789-796.
Dickson, M.A., Hahn W.C, Ino Y, Ronfard, V., Wu, J.Y., Weinberg, R.A., Louis, D.N., Li, F.P., Rheinwald, J.G., 2000. Human keratinocytes that express hTERT and also bypass a p16(INK4a)-enforced mechanism that limits life span become immortal yet retain normal growth and differentiation characteristics. Mol Cell Biol. 20, 1436-47.
EI Ghalbzouri, A., Siamari, R., Willemze, R., Ponec, M., 2008. Leiden reconstructed human epidermal model as a tool for the evaluation of the skin corrosion and irritation potential according to the ECVAM guidelines. Toxicol In Vitro. 22, 1311-20
Emter, R., Ellis, G., Natsch, A., 2010. Performance of a novel keratinocyte-based reporter cell line to screen skin sensitizers in vitro. Toxicol Appl Pharmacol. 245, 281-90
Esser, P.R., Wölfle, U., Dürr, C., von Loewenich, F.D., Schempp, C.M., Freudenberg, M.A., Jakob, T., Martin, S.F., 2012. Contact sensitizers induce skin inflammation via ROS production and hyaluronic acid degradation. PLoS One.7, e41340.
Gober, M.D., Gaspari, A.A., 2008. Allergic contact dermatitis. Curr Dir Autoimmun. 10,1-26
Gotz, C., Pfeiffer, R., Tigges, J., Blatz, V., Jackh, C., Freytag, E. M., Fabian, E., Landsiedel, R., Merk, H. F., Krutmann, J., Edwards, R. J., Pease, C., Goebel, C., Hewitt, N. & Fritsche, E., 2012. Xenobiotic metabolism capacities of human skin in comparison with a 3D epidermis model and keratinocyte-based cell culture as in vitro alternatives for chemical testing: activating enzymes (Phase I). Exp Dermatol 21, 358-63.
Hewitt, N.J., Edwards, R., Fritsche, E., Goebel, C., Aeby, P., Scheel, J., Reisinger, K., Ouedraogo, G., Duche, D., Eilstein, J., Latil, A., Kenny, J., Moore, C., Kuehnl, J., Barroso, J., Fautz, R., Pfuhler, S., 2013. Use of human in vitro skin models for accurate and ethical risk assessment: metabolic considerations. Toxicol Sci. [Epub ahead of print]
Hu, T., Khambatta, Z.S., Hayden, P.J., Bolmarcich, J., Binder, R.L., Robinson, M.K., Carr, G.J., Tiesman, J.P., Jarrold, B.B., Osborne, R., Reichling, T.D., Nemeth, S.T., Aardema, M.J., 2010. Xenobiotic metabolism gene expressiom in the EpiDermin vitro 3D human epidermis model compared to human skin. Toxicol In Vitro. 24, 1450-63
Kaplan, D.H., Igyártó, B.Z., Gaspari, A.A., 2012. Early immune events in the induction of allergic contact dermatitis. Nat Rev Immunol. 12, 114-24
Kimber, I., Dearman, R. J., Scholes, E. W., Basketter, D. A., 1994. The local lymph node assay: developments and applications. Toxicology. 93, 13-31.
Kimber, I., Dearman, R.J. 2002. Allergic contact dermatitis: the cellular effectors. Contact Dermatitis 46, 1-5.
Kollisch, G., Kalali, B.N., Voelcker, V., Wallich, R., Behrendt, H., Ring, J., Bauer, S., Jakob, T., Mempel, M., Ollert, M., 2005. Various members of the Toll-like receptor family contribute to the innate immune response of human epidermal keratinocytes. Immunology 114, 531-541.
McKim, J.M., Keller, D.J., Gorski, J.R., 2010. A new in vitro method for identifying chemical sensitizers combining peptide binding with ARE/EpRE-mediated gene expression in human skin cells. Cutan Ocul Toxicol. 29, 171-92.
McKim, J.M., Keller, D.J., Gorski, J.R., 2012. An in vitro method for detecting chemical sensitization using human reconstructed skin models and its applicability to cosmetic, pharmaceutical, and medical device safety testing. Cutan Ocul Toxicol. 31,292-305
Natsch, A., Emter, R., 2008. Skin sensitizers induce antioxidant response element dependent genes: application to the in vitro testing of the sensitization potential of chemicals. Toxicol Sci. 102, 110-9.
Natsch, A., 2010. The Nrf2-Keap1-ARE toxicity pathway as a cellular sensor for skin sensitizers functional relevance and a hypothesis on innate reactions to skin sensitizers. Toxicol. Sci. 113, 284-92.
Neis, M.M., Wendel, A., Wiederholt, T., Marquardt. Y., Joussen, S., Baron, J.M., Merk, H.F., 2010. Expression and induction of cytochrome p450 isoenzymes in human skin equivalents. Skin Pharmacol Physiol. 23, 29-39
Nickoloff, B.J., 2006. Keratinocytes regain momentum as instigators of cutaneous inflammation.Trends Mol. Med. 12, 102-6
Nestle, F.O., Di Meglio, P., Qin, J.Z., Nickoloff, B.J., 2009. Skin immune sentinels in health and disease. Nat. Rev. Immunol. 9, 679-691.
Ponec, M., Weerheim, A., Kempenaar, J., Mulder, A., Gooris, G.S., Bouwstra, J., Mommaas, A.M., 1997. The formation of competent barrier lipids in reconstructed human epidermis requires the presence of vitamin C. J Invest Dermatol. 109, 348-55.
Ponec, M., Boelsma, E., Weerheim, A., 2000a. Covalently bound lipids in reconstructed human epithelia. Acta Derm. Venereol. 80, 89-93.
Ponec, M., Boelsma, E., Weerheim, A., Mulder, A., Bouwstra, J., Mommaas, M., 2000b. Lipid and ultrastructural characterization of reconstructed skin models. Int. J. Pharm. 203, 211-225.
Ponec, M., Boelsma, E., Gibbs, S., Mommaas, M., 2002. Characterization of reconstructed skin models. Skin Pharmacol. Appl. Skin Physiol. 15 (Suppl. 1), 4-17.
Rasmussen, C., Gratz, K., Liebel, F., Southall, M., Garay, M., Bhattacharyya, S., Simon, N., Vander Zanden, M., Van Winkle, K., Pirnstill, J., Pirnstill, S., Comer, A., Allen-Hoffmann, B.L., 2010. The StrataTest® human skin model, a consistent in vitro alternative for toxicological testing. Toxicol. In Vitro. 24, 2021-9
Saito, K., Miyazawa, M., Nukada, Y., Sakaguchi, H., Nishiyama, N., 2013. Development of an in vitro skin sensitization test based on ROS production in THP-1 cells. Toxicol In Vitro. 27, 857-63
Thakoersing VS, Ponec M, Bouwstra JA.,2010. Generation of human skin equivalents under submerged conditions-mimicking the in utero environment.Tissue Eng Part A. 2010.16,1433-41
Thakoersing, V.S., Gooris, G.S., Mulder, A., Rietveld, M., EI Ghalbzouri, A., Bouwstra, J.A.,2012. Unraveling barrier properties of three different in-house human skin equivalents. Tissue Eng Part C Methods. 2012. 18, 1-11.
van der Veen JW, Pronk TE, van Loveren H, Ezendam J., 2013. Applicability of a keratinocyte gene signature to predict skin sensitizing potential. Toxicol. In Vitro. 27,314-22
Vandebriel, R.J., van Loveren, H., 2010. Non-animal sensitization testing: state-of-the-art. Crit Rev Toxicol.40, 389-404.
Vandesompele, J., De Preter, K., Pattyn, F., Poppe, B., Van Roy, N., De Paepe, A., Speleman, F., 2002. Accurate normalization of real-time quantitative RT-PCR data by geometric averaging of multiple internal control genes. Genome Biol. 18,3
Vocanson, M., Hennino, A., Rozieres, A., Poyet, G., Nicolas, J.F., 2009. Effector and regulatory mechanisms in allergic contact dermatitis. Allergy. 64, 1699-1714.

## Claims

1. In vitro method for predicting the in vivo skin sensitizing activity of a test compound comprising the steps of:
a. Providing a cell culture of immortalised keratinocyte cells,
b. Contacting the cell culture with the test compound,
c. Measuring the expression level of at least the gene OSGIN-1,
wherein it is concluded that the compound has in vivo skin sensitizing activity if the expression level of the gene OSGIN-1 is above a predetermined reference level.

2. In vitro method according to claim 1 wherein the immortalised keratinocyte cells are N/TERT cells.

3. Method according to claims 1 or 2 wherein the cells are comprised in a stratified keratinocyte layer with a competent barrier.

4. Method according to claims 1 - 3 wherein also the expression of the gene GCLM is determined and wherein it is concluded that the compound has in vivo skin sensitizing activity if the expression level of the genes OSGIN-1 and GCLM are above a predetermined reference level.

5. Method according to any one of claims 1 - 4 wherein the predetermined reference level is the level of expression of the genes OSGIN-1 or GCLM when the cell culture is exposed to an irritant.

6. Method according to claim 5 wherein the irritant is SDS or DCB.
